# EUROPEAN PATENT APPLICATION

(11) **EP 3 960 852 A1**
(43) Date of publication of application: **02.03.2022**
(21) Application number: 20192331.5
(22) Date of filing: 24.08.2020
(51) Int. Cl.: C12N 9/12, A61K 38/00, A61K 45/00, A61P 35/00

(54) **PEPTIDES FOR THE TREATMENT OF CANCER**

(71) Applicant: Klinikum rechts der Isar der Technischen Universität München, 81675 München (DE)
(72) Inventor: KONG, Bo, 80809 München (DE)
(74) Representative: Engelhard, Markus

(57) **Abstract**

The present invention relates to peptides having efficacy in the treatment of cancer. Furthermore, the present invention also relates to compositions comprising such peptides and to uses of such peptides and compositions.

## Description

The present invention relates to peptides having efficacy in the treatment of cancer. Furthermore, the present invention also relates to compositions comprising such peptides and to uses of such peptides and compositions.

Pancreatic ductal adenocarcinoma (PDAC) is an extremely aggressive disease. Despite enormous advances in understanding the molecular tumour biology, the overall survival rate of PDAC patients has remained almost unchanged for the past 20 years. Despite its heterogeneity on genetic, histological and clinical levels, oncogenic KRAS mutations (e.g. KRAS^{G12D}) are the driver mutations of the disease. TP53 is also frequently inactivated in PDAC; however, more than one-third of cases maintain wild-type TP53. Tremendous effects have been made to restore p53 function in PDAC; yet, no effective p53-based therapy is translated into the clinic.

There remains a need for efficient treatment approaches that make use of p53. There also is an ongoing need for efficient treatment approaches for cancers other than pancreatic cancer, also making use of a p53-based approach.

In a first aspect, the present invention relates to a peptide having an amino acid sequence NTXIYY (SEQ ID NO:1), wherein X = A (SEQ ID NO:2) or T (SEQ ID NO:3).

In one embodiment, the peptide "comprises" said amino acid sequence, wherein it should however be noted that such embodiment peptide is still limited in its overall length and does not have a length greater than 30 amino acid residues in total, preferably not greater than 25, more preferably not greater than 20, even more preferably not greater than 15, even more preferably not greater than 10, and yet even more preferably not greater than 8 amino acid residues in total.

In one embodiment, said peptide is a hexapeptide, heptapeptide, octapeptide, nonapeptide or decapeptide.

In one embodiment of a peptide according to the present invention, X is A.

In one embodiment, said peptide is a hexapeptide and has an amino acid sequence NTAIYY (SEQ ID NO:2).

In one embodiment, the peptide according to the present invention further comprises an N-terminal protecting group and/or a C-terminal protecting group.

In one embodiment, the N-terminal protecting group has the general formula -C(O)-R, wherein R is selected from the group consisting of H, unsubstituted or substituted alkyls, and unsubstituted or substituted aryls, and wherein the C-terminal protecting group is -NR₁R₂, R₁ and R₂ being independently selected from H and C₁-C₁₀ alkyl, said-NR₁R₂ thus forming an amide group at the C-terminus of said peptide; wherein, preferably, R is selected from the group consisting of methyl, ethyl, propyl, isopropyl, butyl and isobutyl, wherein, more preferably, the N-terminal protecting group is an acetyl group, and wherein, more preferably, the C-terminal protecting group is NH₂.

In one embodiment, the peptide according to the present invention is said peptide is one of the following:
- encapsulated into a liposome;
- linked to or encapsulated into a nanoparticle; or
- encapsulated into an extracellular vesicle, which, preferably is an exosome.

In one embodiment, said peptide is encapsulated into a liposome, and wherein said liposome comprises a phospholipid selected from phosphatidylcholine, phosphatidylethanolamine, phosphatidylinositol, phosphatidylserine, phosphatidic acid, phosphoinositides, phosphatidylinositol monophosphate, phosphatidylinositol bisphosphate, phosphatidylinositol triphosphate, ceramide phosphorylcholine, ceramide phosphorylethanolamine, ceramide phosphoryllipid and mixtures of any of the foregoing; or
said peptide is linked to a nanoparticle, and wherein said nanoparticle is a solid nanoparticle, preferably selected from gold (Au) nanoparticles, silica nanoparticles, and carbon nanotubes; or
said peptide is encapsulated into a nanoparticle and wherein said nanoparticle is a polymeric nanoparticle comprising a polymeric shell surrounding a core, wherein, preferably, said shell comprises one or more of the following polymers: poly(lactic acid), poly(lactic-co-glyclic acid), chitosan, gelatin, poly-alkyl-acyanoacrylate, and mixtures of the foregoing, and said peptide is contained within said core of said polymeric nanoparticle; or
said peptide is encapsulated into an extracellular vesicle, which, preferably is an exosome, and said extracellular vesicle, preferably said exosome is produced from a suitable cell/cell line.

In one embodiment, said liposome additionally comprises a sterol, preferably selected from cholesterol, sitosterol, stigmasterol, stigmastanol, campesterol, fucosterol, brassicasterol, ergosterol, 9,11-dehydroergosterol, daucosterol, and esters of any of the foregoing.

In a further aspect the present invention also relates to a composition comprising a peptide according to the present invention as defined herein, an anti-cancer agent, and a pharmaceutically acceptable carrier.

In one embodiment, said anti-cancer agent is selected from alkylating agents, platin analogues, intercalating agents, antibiotics, inhibitors of mitosis, taxoids, topoisomerase inhibitors, RNA polymerase inhibitors, antimetabolites, wherein, preferably, said anti-cancer agent is coupled to a targetting moiety, in particular an antibody, antibody fragment, a growth factor or growth factor receptor, such as EGF or EGFR, or a fragment of any of the foregoing allowing the selective localisation to a tumour or cancerous tissue.

In one embodiment,
- said alkylating agents are selected from cyclophosphamide, busulfan, carmustine, procarabzine and dacarbazine;
- said platin analogues are selected from oxaliplatin, cisplatin, carboplatin, and satraplatin;
- said intercalating agents are selected from anthracyclins, in particular doxorubicin, daunorubicin, epirubicin, idarubicin; mitoxantron, and amsacrin;
- said inhibitors of mitosis are selected from vincristin, vinblastin, vindesin and vinorelbin;
- said taxoids are selected from paclitaxel, cabazitaxel and docetaxel;
- said topoisomerase inhibitors are selected from camptothecin, topotecan, irinotecan, etoposide and teniposide;
- said RNA polymerase inhibitors are selected from amatoxins, in particular α-amanitin; and
- said antimetabolites are selected from pyrimidine analogues, in particular 5-fluoruracil, gemcitabin and cytarabine; purine analogues, in particular mercaptopurine, 6-thioguanine, azathioprin and fludarabin; and folic acid antagonists, in particular methotrexyate and pemetrexed.

In a further aspect the present invention also relates to a peptide or composition according to the present invention as defined herein, for use as a medicament.

In a further aspect the present invention also relates to a peptide or composition according to the present invention as defined herein, in a method for preventing development or growth of a cancer tumour in a patient, and/or in a method of treating a cancer tumour in a patient, said cancer tumour being characterised by having a wildtype-version of the *TP53* gene, and not having a mutated version of the *TP53* gene, wherein, in said method, said peptide or composition is administered to a patient having or suspected of having or developing a cancer tumour.

As used herein, the term "having a wildtype-version of the *TP53* gene, and not having a mutated version of the *TP53* gene", when used in the context of a cancer tumour or cancerous tissue, is meant to refer to a scenario wherein the majority of cancer cells within said tumour or said tissue, preferably all of said cancer cells within said tumour or said tissue, are characterised by wildtype *TP53* and do not have a mutated version of *TP53.*

In one embodiment, said cancer tumour is a pancreatic cancer tumour, a breast cancer tumour, a colon cancer tumour or a lung cancer tumour, wherein, preferably, said cancer tumour is a pancreatic cancer tumour which is selected from pancreatic adenocarcinoma tumour, pancreatic acinar cell carcinoma tumour, and pancreatic cystadenocarcinoma tumour, wherein, more preferably, said cancer tumour is a pancreatic ductal adenocarcinoma (PDAC) tumour.

In a further aspect the present invention also relates to a peptide according to the present invention as defined herein, in a method of sensitising a cancer tumour to the action of an anti-cancer agent in a patient, wherein, in said method, said peptide is administered to a patient having or suspected of having or developing a cancer tumour, and wherein said anti-cancer agent is administered to said patient concomitantly with said administration of said peptide.

The present invention also relates to a method for preventing development or growth of a cancer tumour in a patient and/or to a method of treating a cancer tumour in a patient, said cancer tumour being characterized by having a wild type-version of the *TP53* gene, and not having a mutated version of the *TP53* gene, wherein, in said method, said peptide is administered to a patient having or suspected of having or developing a cancer tumour.

The present invention also relates to the use of a peptide or composition, as defined above, for the manufacture of a medicament in a method for prevention development or growth of a cancer tumour in a patient, and/or in a method of treating a cancer tumour in a patient, said cancer tumour being characterized by having a wild type-version of the *TP53* gene, and not having a mutated version of the *TP53* gene, wherein, in said method, said peptide or composition is administered to a patient having or suspected of having or developing a cancer tumour.

In any of the aforementioned aspects, the cancer tumour is as defined herein.

In a further aspect, the present invention also relates to a method of sensitizing a cancer tumour to the action of an anti-cancer agent in a patient, wherein, in said method, said peptide is administered to a patient having or suspected of having or developing a cancer tumour, and wherein said anti-cancer agent is administered to said patient concomitantly with said administration of said peptide.

As used herein, the term "peptide" as used herein, is meant to refer to a polymer of amino acid residues wherein the total number of amino acid residues does not exceed 30 amino acids. In one embodiment, a peptide is limited in its total number of amino acid residues to 25 or less, preferably 20 or less, more preferably 15 or less, even more preferably 10 or less, and even more preferably 8 or less. It should be noted that the minimum number of amino acid residues in a peptide in accordance with the present invention is 6.

The term "a peptide having an amino acid sequence" is meant to refer to a peptide that comprises such an amino acid sequence. In one embodiment, such term is however, meant to refer to a peptide that consist of such an amino acid sequence. The term "consists of" implies that there may not be any further amino acid residues attached; however, such term should be understood to still allow for the presence of other non-amino-acid entities being optionally attached, such as one or several protecting group(s), post-translationally modifying groups, such as PEG, etc.

The terms "targetting moiety" or "targetting agent", as used herein, refer to species that will selectively localize in a particular tissue or region of the body. The localization is mediated by specific recognition of molecular determinants, molecular size of the targeting agent or conjugate, ionic interactions, hydrophobic interactions and the like. Other mechanisms of targeting an agent to a particular tissue or region are known to those of skill in the art. As an example, one such targetting moiety may be an anti-EpCAM-antibody, selectively binding to and targetting epithelial cell adhesion molecule (EpCAM) which is overexpressed in epithelial cancers, and thus targetting specifically cancerous tissue and cells overexpressing epithelial cell adhesion molecule (EpCAM). Any entity that is attached to a targetting moiety will thus also be targetted to specifically cancerous tissue and cells.

As used herein, "anti-cancer agent" means any agent useful to combat cancer including cytotoxins and agents such as antimetabolites, alkylating agents, anthracyclines, antibiotics, antimitotic agents, procarbazine, hydroxyurea, asparaginase, corticosteroids, interferons and radioactive agents. Also encompassed within the scope of the term "anti-cancer agent," are conjugates of proteins with anti-tumor activity, e.g. TNF-α. Conjugates include, but are not limited to those formed between a therapeutic protein and a peptide of the invention.

As used herein, "a cytotoxin or cytotoxic agent" means any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracinedione, mitoxantrone, mithramycin, actinomycin D, 1-dehydrotestosterone, glucocorticoids, procaine, tetracaine, lidocaine, propranolol, and puromycin and analogs or homologs thereof. Other toxins include, for example, ricin, CC-1065 and analogues, the duocarmycins. Still other toxins include diphtheria toxin, and snake venom (e.g., cobra venom).

As used herein, "pharmaceutically acceptable carrier" includes any material, which when combined with the conjugate retains the activity of the conjugate activity and is non-reactive with the subject's immune system. Examples include, but are not limited to, any of the standard pharmaceutical carriers such as a phosphate buffered saline solution, water, emulsions such as oil/water emulsion, and various types of wetting agents. Other carriers may also include sterile solutions, tablets including coated tablets and capsules. Typically such carriers contain excipients such as starch, milk, sugar, certain types of clay, gelatin, stearic acid or salts thereof, magnesium or calcium stearate, talc, vegetable fats or oils, gums, glycols, or other known excipients. Such carriers may also include flavor and color additives or other ingredients. Compositions comprising such carriers are formulated by well known conventional methods.

As used herein, "administering" means, inter alia, oral administration, administration as a suppository, topical contact, intravenous, intraperitoneal, intramuscular, intralesional, intranasal or subcutaneous administration, intrathecal administration, or the implantation of a slow-release device *e.g.,* a mini-osmotic pump, to the subject.

A "concomitant" administration is meant to refer to an administration wherein two agents are administered to a patient together in a single administration or such that over a defined period of time, a patient is exposed to the action of both agents. The two agents may be administered within the same dosage unit or in separate dosage units, which may be taken together or separately.

As used herein, the term "extracellular vesicle" refers to intracellularly formed vesicles which are subsequently secreted. They may be viewed as a "nanosphere" or "nanospheres" with a bilayered lipid membrane. Depending on the size of such extracellular vesicles, these may be further classified into
- exosomes (which typically have an average diameter in the range of from 30nm to 50nm),
- microvesicles or ectosomes (which typically have an average diameter in the range of from 50nm to 1µm), and
- apoptotic bodies (which typically have an average diameter in the range of from 50nm to 1µm).

Of particular interest in the context of the present invention are exosomes and microvesicles which may be used for encapsulation of a peptide or peptides according to the present invention.
One of the most useful properties of exosomes is their capability to cross barriers such as cytoplasmic membranes and/or the blood/brain barrier. This makes them particularly amenable to be used as therapeutic delivery carriers. Exosomes have great potential of being a drug carrier due to their natural material transportation properties, intrinsic long-term circulatory capability, and excellent biocompatibility, which are suitable to deliver a variety of chemicals, proteins, nucleic acids and gene therapeutic agents.

The production of extracellular vesicles, and in particular, of exosomes, and their manufacture as well as loading with drugs is known to a person skilled in the art and has for example been described in Luan et al., Acta Pharmacologica sinica, 2017; vol.38; pp. 754 -763; Bunggulura et al. J. Nanobiotechnol., 2018; vol. 16; pp. 81 ff; and Liu et al. Theranostics, 2019, vol.9(4); pp. 1015 - 1028.

Furthermore in the present application, various peptide sequences are designated as follows:
SEQ ID NO: 1 NTXIYY, wherein X may be A or T;
SEQ ID NO:2 NTAIYY
SEQ ID NO:3 NTTIYY
SEQ ID NO:4 PTTIYY
SEQ ID NO:5 NTAIYA
SEQ ID NO:6 TAIYY
SEQ ID NO:7 LEHTTLRKVT ANTAIYYDPN PQSTVVAEDQ (stretch of Polr2a sequence from Mus musculus)
SEQ ID NO:8 LEHTTLRKVT ANTAIYYDPN PQSTVVAEDQ (stretch of Polr2a sequence from Homo sapiens)
SEQ ID NO:9 TX1X2X3X4, wherein X1 may be any amino acid, X2 may be I or L, X3 may be Y or F, and X4 may be Y or F.

Furthermore, the present invention is now further described by reference to the figures, wherein

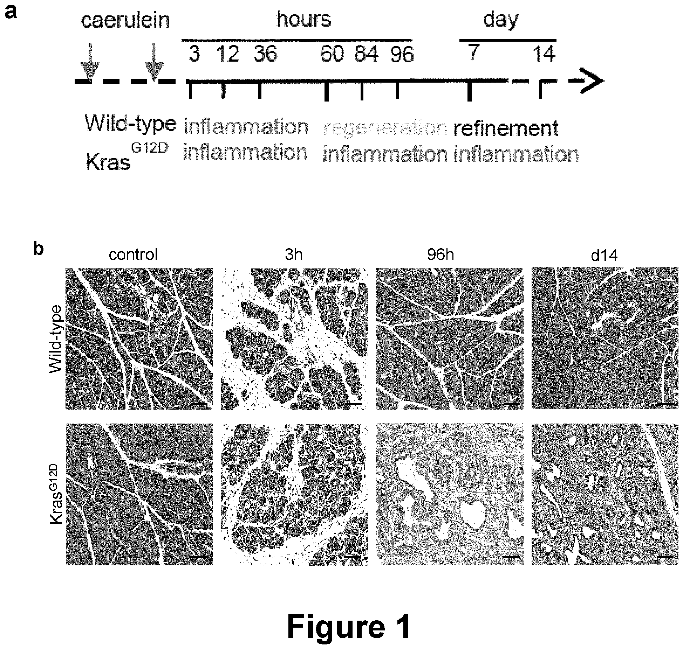

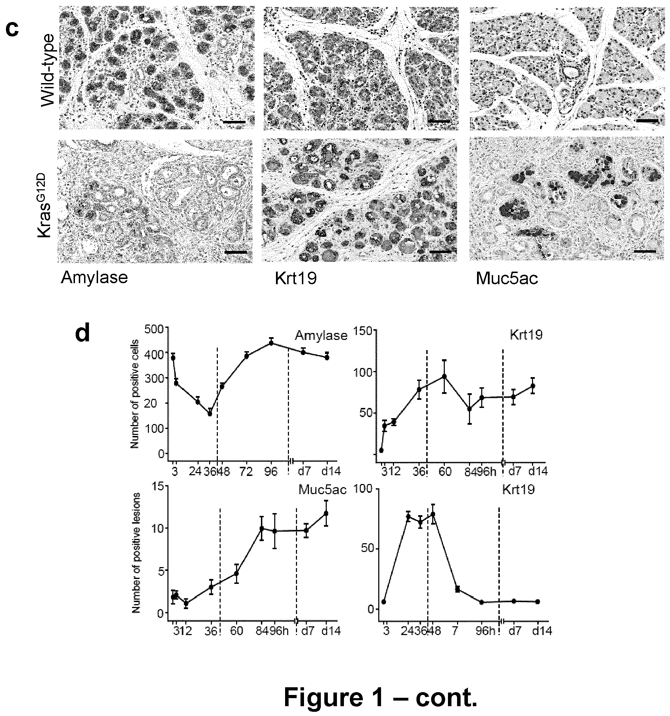

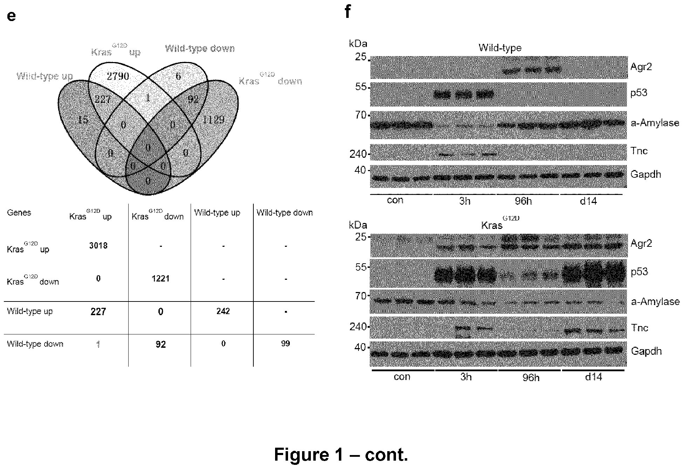

Figure 1 shows a model of caerulein-induced pancreatitis and various aspects in relation thereto. More specifically, figure 1 shows the following:
(**a**) Wild-type (WT) or Kras^{G12D} mice were sacrificed between 3 hours and 14 days after caerulein-induced acute pancreatitis; (**b**) Representative H&E-stained sections with histological features of the inflammation, regeneration and refinement phases in WT pancreata, but of the persistent inflammation and early carcinogenesis in Kras^{G12D} pancreata (scale bars: 50 µm); (**c**) Representative IHC pictures show a-amylase-, Krt19- and Muc5ac-positive cells in the different phases of inflammation in WT and Kras^{G12D} pancreata (right panel); scale bars: 50 µm. (**d**) Quantitative data show time-dependent changes in the number of a-amylase and Krt19-positive cells in WT pancreata (left); and Muc5ac- of Krt19-positive cells in Kras^{G12D} pancreata (right); (**e**) Venn diagram shows the number of genes up- or down-regulated in WT or Kras^{G12D} pancreata by microarray analysis; (**f**) Western-blot analysis demonstrates levels of Agr2, p53, a-amylase and Tnc and in WT or Kras^{G12D} pancreata 3 hours, 96 hours and 14 days after caerulein treatment.

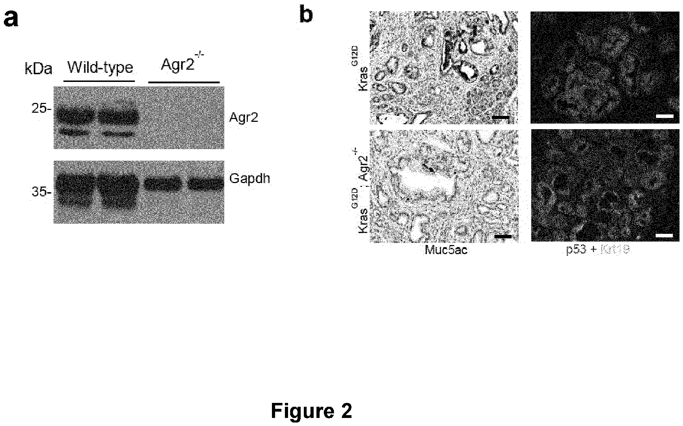

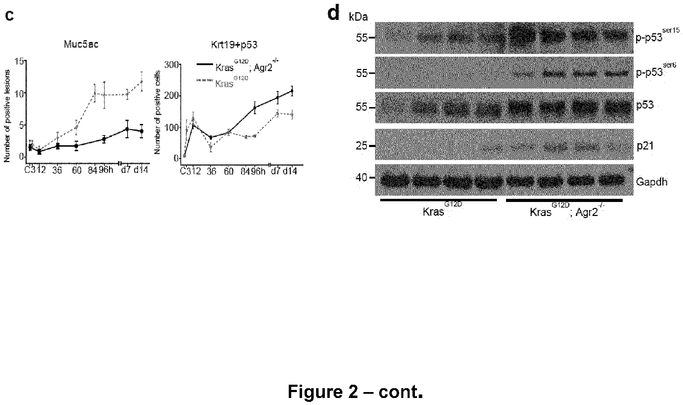

Figure 2 shows the results of a mouse model in which Agr2 is specifically ablated in the pancreatic epithelial cells. More specifically, figure 2 shows:
(**a**) Western-blot analysis shows levels of Agr2 in WT and Agr2^{-/-} pancreata; (**b,c**) Quantitative data show time-dependent changes in the number of Muc5ac- and p53-positive cells in Kras^{G12D} and Kras^{G12D}; Agr2^{-/-} pancreata; Representative IHC or IF pictures show Muc5ac staining and p53 and Krt19 double-positive cells (scale bars: 50 µm); (**d**) Western-blot analysis demonstrates levels of p-p53^{ser15}, p-p53^{ser6}, p53 and p21 in Kras^{G12D} and Kras^{G12D}; Agr2^{-/-} pancreata 96 hours after caerulein treatment.

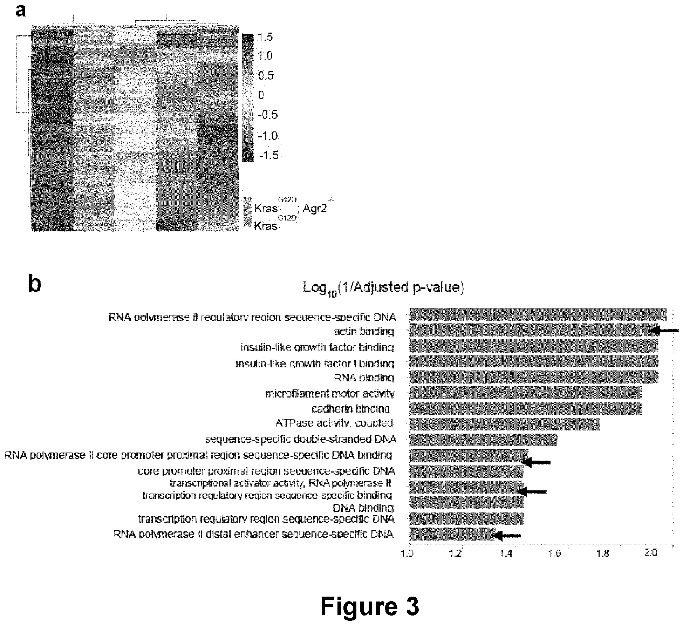

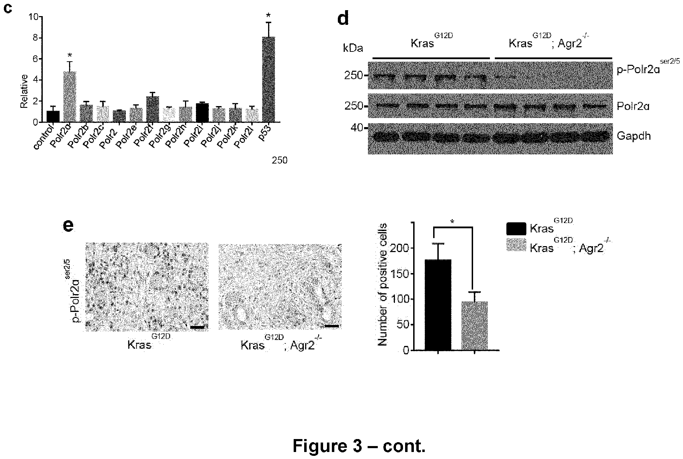

Figure 3 shows the possible role that Agr2 has on the p53 pathway. More specifically, figure 3 shows:
(**a**) A heatmap illustrates the dysregulated genes between Kras^{G12D} and Kras^{G12D}; Agr2^{-/-} pancreata 96 hours after caerulein treatment; (**b**) Gene Ontology (GO) term analysis shows enriched biological processes correlated with the p53 activation after Agr2 ablation in Kras^{G12D} pancreata; (**c**) Quantitative PCR data show relative mRNA expression of subunits of RNA polymerase II (Polr2a-2i) and p53 in Kras^{G12D} and Kras^{G12D}; Agr2^{-/-} pancreata 96 hours after caerulein treatment; (**d**) Western-blot analysis demonstrates levels of p-Polr2a^{ser2/5} and Polr2a in Kras^{G12D} and Kras^{G12D}; Agr2^{-/-} mice 96 hours after caerulein treatment; (**e**) Representative IHC picture shows p-Polr2a^{ser2/5}-positive cells in Kras^{G12D} and Kras^{G12D}; Agr2^{-/-} pancreata 96 hours after caerulein treatment (left, scale bar: 50 µm).

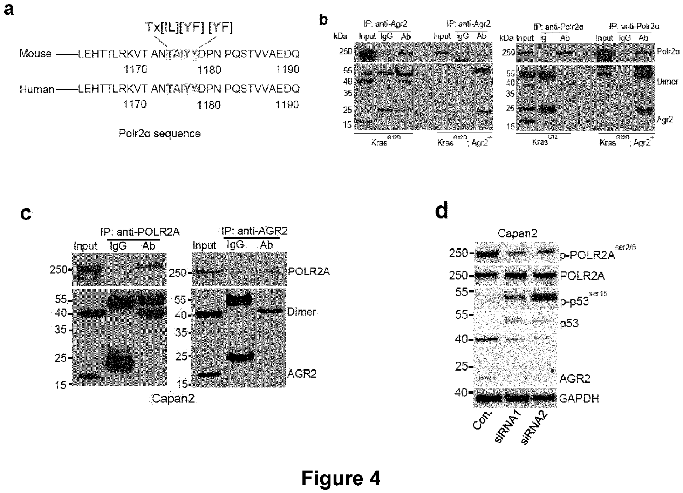

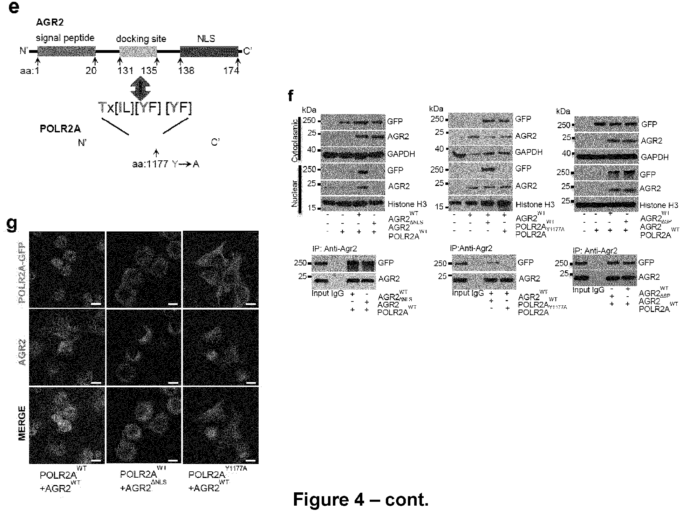

Figure 4 shows that Agr2 directs the POLR2A nuclear import. More specifically, figure 4 shows:
(**a**) Protein sequence of human and mouse Polr2a contains a peptide-binding site for Agr2; (**b**) The results of co-IP assay demonstrate the binding between Agr2 and Polr2a in bulk pancreatic tissues; (**c**) The results of co-IP assay demonstrate the binding between AGR2 and POLR2A in human PDAC cells (Capan2); (**d**) Western blot analysis shows the levels of p-POLR2A^{ser2/5}, p53, p53^{ser15} and AGR2 in Capan2 and HPAC cells after AGR2 siRNA transfection; one of three independent experiments is shown; (**e**) The diagram depicts the signal peptide (SP: aa: 1-20), the presumed docking site of Polr2a (aa: 131-135) and the NLS (aa: 138-174); (**f**) Western-blot analysis shows the expression of exogenous POLR2A (fused with GFP) and AGR2 in the cytosolic and nuclear fraction of HEK293 cells co-transfected with different AGR2 and POLR2A expression vectors (upper panel); the results of co-IP assay demonstrate the binding between exogenous AGR2 and POLR2A in HEK293 cells; one of three independent experiments is shown; (**g**) Immunofluorescence analysis showing the localization of POLR2A (labelled by GFP) in HEK293 cells co-transfected with different AGR2 and POLR2A expression vectors, DAPI: 4',6-diamidino-2-phenylindole; scale bars: 20 µm.

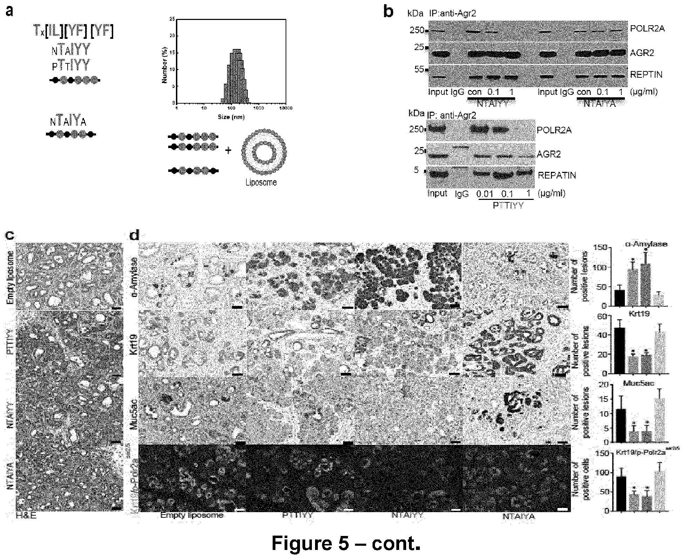

Figure 5 shows that liposome-encapsulated peptides prevent pancreatic carcinogenesis in vivo. More specifically, figure 5 shows:
(**a**) Schematic model of generating liposome-encapsulated bioactive (inactive) hexapeptides (left panel); Bar chart illustrates the size distribution of liposomes (right panel); (**b**) The results of co-IP demonstrate that NTAIYY (SEQ ID NO:2) and PTTIYY (SEQ ID NO:4) dose-dependently block the binding of AGR2 to POLR2A in Capan2 cells; (**c**) Representative H&E-stained sections show the histology of controls-(empty liposomes and NTAIYA (SEQ ID NO:5)) or hexapeptide-treated (NTAIYY (SEQ ID NO:2) and PTTIYY (SEQ ID NO:4)) Kras^{G12D} pancreata, scale bars: 100 µm; quantitative analysis shows the number of α-amylase-, Krt19- Muc5ac-, p53- and Krt19/p-Polr2a^{ser2/5}-positive cells in hexapeptides and controls-treated pancreata; *: <0.05, unpaired t-test as compared to empty liposomes-treated samples.

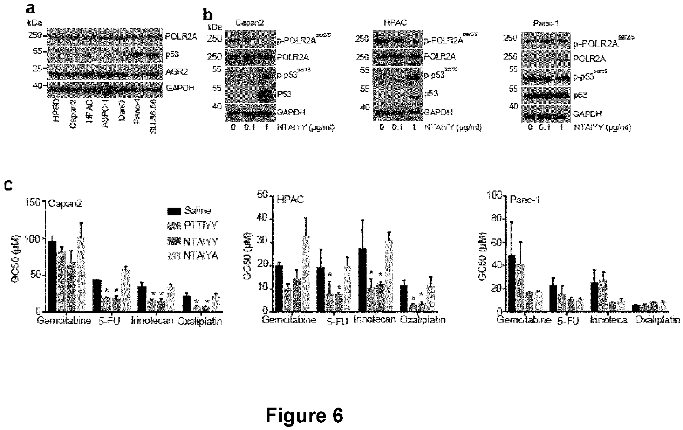

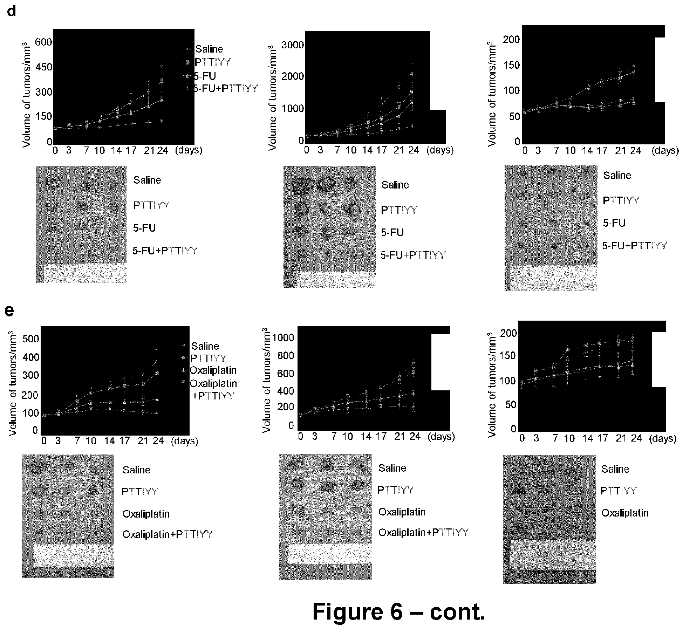

Figure 6 shows that peptides in accordance with the present invention increase the cytostatic effect of chemotherapeutic agents. More specifically, figure 6 shows:
(**a**) Western-blot analysis demonstrates the expression of POLR2A, p53 and AGR2 in human PDAC cell lines and HPED cells; (**b**) Western-blot analysis shows the levels of p-POLR2A^{ser2/5}, POLR2A, p-p53^{ser15} and p53 in Capan2, HPAC and Panc1 cells treated with bioactive hexapeptide (NTAIYY (SEQ ID NO:2)) or controls; (**c**) Bar graphs show the GI50 (growth inhibition 50%) of Gemcitabine, 5-FU, Irinotecan and Oxaliplatin in PDAC cell lines with either wild-type (Capan2 and HPAC cells) TP53 or mutated (Panc1 cells) TP53 in the presence of bioactive hexapeptides (NTAIYY (SEQ ID NO:2) and PTTIYY (SEQ ID NO:4)) or controls (empty liposome and NTAIYA (SEQ ID NO:5)); The data represent three independent experiments; (**d-e**) The results of chemotherapy assay using the subcutaneous xenografts demonstrate the cytostatic effect of 5-FU and Oxaliplatin in combination with either bioactive hexapeptide (PTTIYY (SEQ ID NO:4)) or control (Saline); *: <0.05, unpaired t-test.

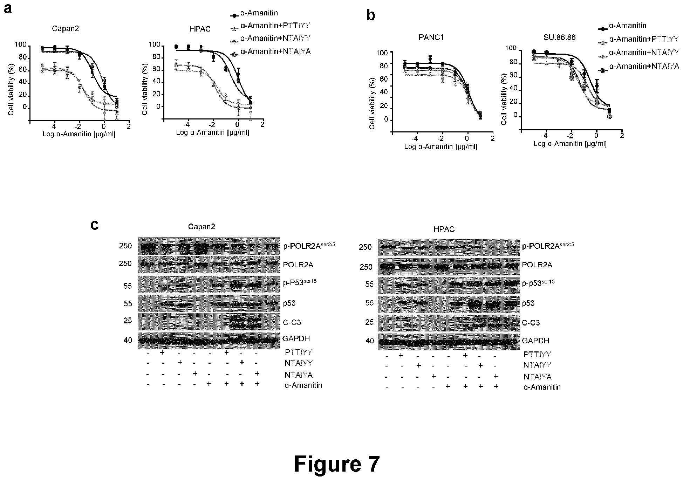

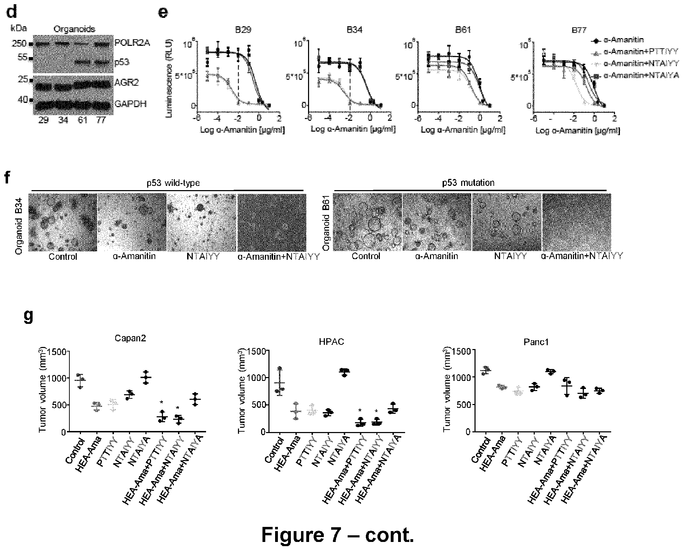

Figure 7 shows that the peptides according to the present invention intensify the cytotoxicity of α-Amanitin in human PDAC cell lines. More specifically, figure 7 shows:
(**a-b**) The results of MTT assay demonstrate the dose-dependent changes in the cell viability of Capan2, HAPC, Panc1 and Su86.86 cells treated with bioactive (inactive) dexapeptides and α-Amanitin; (**c**) Western-blot analysis demonstrates the levels of p-POLR2A^{ser2/5}, POLR2A, p-p53^{ser15}, p53 and cleaved-caspase3 (C-C3) in Capan2 and HAPC cells treated with bioactive (inactive) dexapeptides and α-Amanitin; One of three similar independent experiments is shown; (**d**) Western-blot analysis demonstrates the expression of POLR2A, p53 and AGR2 of four patient-derived PDAC organoids; (**e**) The results of MTT assay demonstrate the dose-dependent changes in the cell viability of four patient-derived PDAC organoids treated with bioactive (inactive) dexapeptides and α-Amanitin; The data represents three independent experiments; (**f**) Representative phase-contrast pictures show the morphology of patient-derived PDAC organoids treated with control, α-Amanitin, bioactive dexapeptide and both; (**g**) Bar graphs show the volume of PDAC xenografts (Capan2, HPAC and Panc1 cells) in mice under the treatment of Ama-HEA125 and bioactive (inactive) hexapeptides; *: <0.05, unpaired t test.

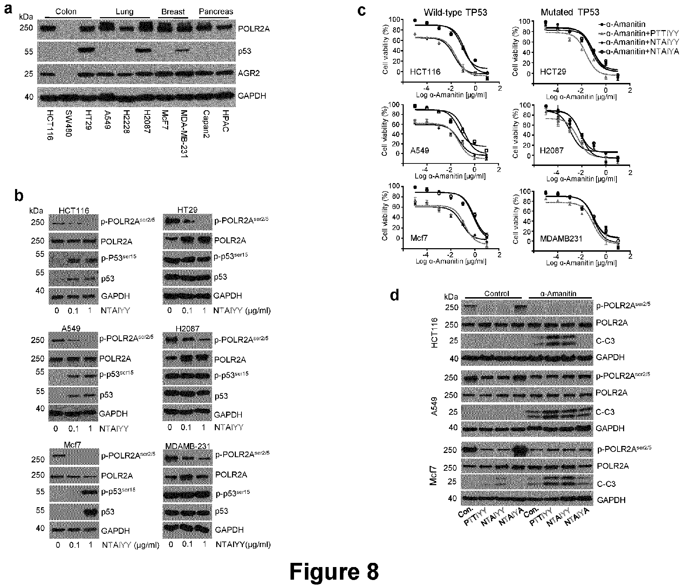

Figure 8 shows that the peptides according to the present invention intensify also the cytotoxicity of α-Amanitin in other human cancer entities. More specifically, figure 8 shows:
(**a**) Western-blot analysis demonstrates the expression of POLR2A, p53 and AGR2 in human colon, breast and lung cancer cell lines; (**b**) Western-blot analysis shows the levels of p-POLR2A^{ser2/5}, POLR2A, p-p53^{ser15} and p53 in different human cancer cell lines treated with bioactive hexapeptide (NTAIYY (SEQ ID NO:2)) or controls; one of three similar experiments is shown; (**c**) The results of MTT assay demonstrate the dose-dependent changes in the cell viability of six human cancer cell lines treated with bioactive (inactive) hexapeptides and α-Amanitin; The data represent three independent experiments; (**d**) Western-blot analysis demonstrates the levels of p-POLR2A^{ser2/5}, POLR2A and cleaved-caspase3 (C-C3) in different human cancer cell lines treated with bioactive hexapeptide (NTAIYY (SEQ ID NO:2)) or controls; one of three similar experiments is shown.

The invention is now further described and exemplified by reference to the following examples which are given to illustrate, not to limit the present invention:

### EXAMPLES

### Example 1 - Material and Methods

The peptides were prepared using a solid phase peptide synthesis method. In brief, polystyrene with surface modification of 4-hydroxybenzyl alcohol was used as the support resin, and 9-fluorenylmethyloxycarbonyl (Fmoc) was used as the base-labile protecting group. According to the peptide sequence, the amino acids were conjugated to the resin through alternative Fmoc deprotection and amide bond coupling. Afterwards, free peptides were obtained after cleavage of the ester linkage to the resin by trifluoroacetic acid. After purification, the peptides were analyzed using reversed-phase-high performance liquid chromatography (RP-HPLC). The purity of all peptides is above 95%.

Hexapeptide-loaded liposomes were prepared using the thin-film dispersion and hydration method. 100 mg of soybean lecithin and 20 mg of cholesterol were dissolved in 20 mL of trichloromethane. 10.0 mg of the custom-made peptide were dissolved in 20 mL of methanol upon a short ultrasonication in a water bath. The obtained solutions of soybean lecithin and hexapeptides were mixed in a round-bottom flask, which was subjected to evaporation in a rotary evaporator at 40 °C to form dry lipid film upon vacuum drying. Subsequently, 20 mL of 5% w/v sucrose purified by passing a syringe membrane (0.22 µm) was added to hydrate the lipid film. After ultrasonication in a water bath, the obtained solution was extruded by passing polycarbonate membranes with pore sizes of 800 nm, 400 nm, 200 nm successively using the extruder (Avanti^{®} Mini-Extruder; Alabama, USA). The peptide-loaded liposomes were purified and concentrated by ultrafiltration (MWCO 100 kDa Millipore, USA). The encapsulated peptide concentration was then determined by reversed-phase high-performance liquid chromatography (RP-HPLC). Empty liposomes without peptide were prepared using the same protocol.

Characterisation: The hydrodynamic size and size distribution were measured at 25 °C using dynamic light scattering (Zetasizer ZS90, Malvern). The average hydrodynamic size in diameter and polydispersity index (PDI) of hexapeptide-loaded liposomes is 179.9 nm and 0.137 (**Figure 5a**). The encapsulation efficiency of the peptide and drug loading in the liposomes are 52.8% and 4.4%, respectively.

### Example 2 - Results

### Agr2 is induced in PanIN lesions and proliferative acinar cells in organ regeneration

Recently, the present inventors characterised the natural course of caerulein-induced pancreatitis in wild-type (C57BL/6J) mice with a high temporal resolution and defined three distinctive phases termed inflammation, regeneration and refinement (**Figure 1a, 1b**). In the presence of oncogenic Kras^{G12D} (p48^{Cre/+}; LSL_Kras^{G12D/+}, hereafter referred to as 'Kras^{G12D}'), there was a persistent inflammatory phase with the appearance of pre-neoplastic lesions including acinar-to-ductal metaplasia (ADM) and PanIN lesions(**Figure 1a, 1b**)³. Under physiological circumstances, ADM is a reversible metaplastic process in which acinar cells transiently adopt a ductal phenotype with the capability of redifferentiating into acinar cells. In the context of Kras^{G12D} and pancreatitis, however, ADM lesions further progress into PanIN lesions. To study this process, the present inventors assessed the dynamic alternation in the number of acinar cells (labelled by α-Amylase), ADM (marked by Krt19) and PanIN (labelled by Muc5ac) lesions in both mouse strains (**Figure 1c**). This analysis revealed that the acquisition of PanIN phenotype in ADM lesions took place at the same time frame when the proliferative acinar cells were massively detected in wild-type mice (regeneration phase, **Figure 1d**). However, in ductal ligation-induced pancreatitis, no regeneration in the acinar compartment was observed in wild-type mice, and no PanIN lesion was detected in Kras^{G12D} mice. These data imply that the PanIN formation in early carcinogenesis and the acquisition of proliferative phenotype in acinar cells during organ regeneration may share specific molecular machinery. To address this, the present inventors compared microarray-based transcriptome data of bulk pancreatic tissues from wild-type and Kras^{G12D} mice (GSE65146). This analysis revealed that the transcriptional profiles of Kras^{G12D} samples were rather similar to inflammatory samples in wild-type mice. There were 319 transcripts which shared the same expression pattern (e.g. α-Amylase or tenascin C (Tnc)), that is consistently increased/decreased in both inflammatory samples of wild-type mice and Kras^{G12D} samples (**Figure 1e**). Nevertheless, there was only one gene Agr2 that is increased in the regeneration samples of wild-type mice and Kras^{G12D} mice. Western blot analysis confirmed this change also on protein levels (**Figure 1f**). As Agr2 is a putative p53 inhibitor⁹, the present inventors observed that p53 was indeed suppressed in the regeneration phase of wild-type mice and transiently inhibited at 96 hours (corresponding to the regeneration phase) in Kras^{G12D}. These data suggested that Agr2 may be functionally relevant to PanIN development and organ regeneration.

### Example 3

### Loss of Agr2 inhibits PanIN formation with p53 activation in Kras^{G12D} mice

To test this, the present inventors first generated p48^{Cre/+}; Agr2^{flox/flox} (hereafter referred to as 'Agr2^{-/-}') in which Agr2 is specifically ablated in all pancreatic epithelial cells. Loss of Agr2 in the pancreatic epithelium was confirmed by western-blot analysis (**Figure 2a**). Indeed, the Agr2 ablation partially delayed organ regeneration in wild-type mice after caerulein administration (data not shown). Next, the present inventors generated p48^{Cre/+}; LSL_Kras^{G12D/+}; Agr2^{flox/flox} (hereafter referred to as 'Kras^{G12D}; Agr2^{-/-}') and treated them with caerulein. Here, the Agr2 ablation dramatically inhibited PanIN formation (as exemplified by quantitative analysis of Muc5ac staining) and activated p53 in ADM lesions (as illustrated by quantitative analysis of double-staining for p53 and Krt19, **Figure 2b****,** **2c**). Western blot analysis confirmed this p53 activation (together with a downstream marker: p21) in bulk pancreatic tissues after caerulein treatment (**Figure 2d**). Also, Agr2 depletion led to phosphorylation of the Ser-15/6 site of p53, mimicking a DNA-damaging signal.

### Example 4

### Loss of Agr2 decreases the activity of RNA polymerase II

To investigate the underlying mechanism responsible for p53 activation in the absence of Agr2, the present inventors compared transcriptional profiles of Kras^{G12D}; Agr2^{-/-} to Kras^{G12D} pancreata. Using 0.01 as the statistical cut-off for the false discovery rate, 437 genes were found to be differentially expressed. Here, p53 is also elevated on the mRNA levels after Agr2-ablation. Among these, the present inventors identified forty-eight genes which are positively associated with p53 expression (**Figure 3a**). To explore the functional relevance, the present inventors analysed Gene Ontology (GO) terms using the 48 preselected genes (http://amp.pharm.mssm.edu/Enrichr/). This analysis revealed that 15 GO terms were over-represented (**Figure 3b**); the most relevant GO terms are biological processes linked with RNA polymerase II (RNAPII)-mediated DNA binding. These data suggest that Agr2 affects the p53 pathway by interfering with the RNAPII activity. RNAPII is a multiprotein complex containing 12 subunits (Polr2a-2i). Notably, the present inventors observed that Polr2a was increased at the transcriptional level (**Figure 3c**); however, its activated form (phosphorylation of the Ser-2/5 site) is significantly decreased (**Figure 3d**). Further immunohistochemistry analysis confirmed this by showing a devoid of nuclear staining of p-Polr2a^{ser2/5} in Kras^{G12D}; Agr2^{-/-} samples (**Figure 3e**).

### Example 5

### AGR2 directs the POLR2A nuclear import

By screening human and mouse proteomes, it is observed that both human and mouse Polr2a contains a single TAIYY motif (SEQ ID NO: 6) (a consensus binding site of Tx[IL][YF][YF], (SEQ ID NO: 9) aa1173-1177) for Agr2 binding (**Figure 4a**). The results of co-immunoprecipitation (co-IP) experiments confirmed this physical binding between endogenous Agr2 and Polr2a in both mouse tissues and human PDAC cells (**Figure 4b, 4c**). The binding of AGR2 to POLR2A is also crucial for the activity of RNAPII in human PDAC cells because AGR2 downregulation (by siRNA transfection) reduces the phosphorylation levels of Ser2/5 residues (p-POLR2A^{ser2/5}), but not the POLR2A expression (**Figure 4d**). POLR2A, the catalytic subunit of RNAPII, is imported into the nucleus and recruited to gene promoters in a hypo-phosphorylated state. Within the active transcription cycle, POLR2A is sequentially phosphorylated by CDK7 and CDK9 (cyclin-dependent kinase 7 and 9) in the nucleus. Since POLR2A itself contains no nuclear localization signal (NLS), it requires an additional NLS-containing factor to mediate its nuclear import in eukaryotic cells. By analyzing the full-length protein sequence of AGR2 (http://nls-mapper.iab.keio.ac.jp/cgi-bin/NLS_Mapper_form.cgi), the present inventors noticed that AGR2 contains an NLS at its C-terminus (aa 138-174, **Figure 4e**). Since POLR2A itself is devoid of NLS, it may require the function of AGR2 to mediate its nuclear import. To test this, based on an expression vector containing human wild-type full-length AGR2 cDNA (AGR2^{WT}), the present inventors generated AGR2-expressing vectors either with the NLS deletion (del138-174 aa, AGR2_{Δ}^{NLS}) or with the signal peptide (SP) deletion (del1-20 aa, as a control, AGR2^{ΔSP}). Also, based on a vector containing human full-length POLR2A cDNA fused with a GFP (green fluorescent protein) tag at the C-terminus (POLR2A^{WT}), a mutant POLR2A was constructed in which the Tyrosine-1177 was replaced by alanine to create the TAIYA motif (POLR2A^{Y1177A}). As such, HEK293 cells were transiently co-transfected with AGR2 and POLR2A vectors. Indeed, the intact function of AGR2 is crucial for directing POLR2A nuclear import; the deletion of NLS on AGR2 abolishes this function without affecting the binding affinity between AGR2 and POLR2A (**Figure 4f, 4g**). Moreover, the Y-A mutation at position 1177 attenuates the ability of POLR2A to bind AGR2; hence, it blunts its nuclear import (**Figure 4f, 4g**). However, the deletion of SP on AGR2 did not have these effects (**Figure 4f, 4g**).

### Example 6

### Liposome-encapsulated hexapeptides prevent Kras^{G12D}-driven pancreatic carcinogenesis in vivo

The present inventors hypothesize that a small peptide mimicking the consensus binding site of Tx[IL][YF][YF] could competitively disrupt the binding between AGR2 and POLR2A by blocking the docking site on AGR2. To test this, the present inventors synthesized a hexapeptide (NTAIYY (SEQ ID NO:2)) according to the endogenous protein sequence of POLR2A. The Y-A mutation at position 6 was introduced to generate the inactive control hexapeptide (NTAIYA (SEQ ID NO:5)). Besides, the present inventors included a previously described hexapeptide (PTTIYY (SEQ ID NO:4)), which is known to precipitate the endogenous AGR2 *in vitro,* as a positive control. To enable its clinical application, the present inventors developed a liposome-encapsulated form of these hexapeptides (**Figure 5a**). The Co-IP results revealed that NTAIYY (SEQ ID NO:2) and PTTIYY (SEQ ID NO:4) inhibited the binding of POLR2A to AGR2 in a dose-dependent manner in PDAC cells (**Figure 5b**). However, the inactive hexapeptide (NTAIYA (SEQ ID NO:5)) had no such effect. Since the interaction between REPTIN and AGR2 is not mediated by the consensus binding site, the binding between REPTIN and AGR2 was not affected under the treatment of NTAIYY (SEQ ID NO:2) and PTTIYY (SEQ ID NO:4), indicating the specificity of both hexapeptides. These data demonstrate that both hexapeptides (NTAIYY (SEQ ID NO:2) and PTTIYY (SEQ ID NO:4)) are bioactive in that they displace POLR2A by competitively blocking the docking site on AGR2. To test the impact of two bioactive hexapeptides on early pancreatic carcinogenesis *in vivo,* the present inventors treated Kras^{G12D} mice with three doses of intravenous hexapeptides (NTAIYY (SEQ ID NO:2) and PTTIYY (SEQ ID NO:4), 8 µg/kg) or controls (empty Liposome and NTAIYA (SEQ ID NO:5)) after caerulein injection. Compared to controls, both NTAIYY (SEQ ID NO:2) and PTTIYY (SEQ ID NO:4) administrations resulted in a decreased number of PanIN lesions (stained by Muc5ac), but an increased amount of intact acinar cells (stained by α-Amylase, **Figure 5c, 5d**). Double staining for Krt19 and p-Polr2a^{ser2/5} revealed a decline in Polr2a activity in bioactive hexapeptides-treated Kras^{G12D} pancreata, approving their specific effects *in vivo* (**Figure 5c, 5d**).

### Example 7

### Bioactive hexapeptides increase the cytostatic effect of chemotherapeutic agents

The present inventors went on to test if bioactive hexapeptides affect the chemosensitivity of PDAC cell lines to chemotherapeutic agents that are routinely used, including Gemcitabine, 5-FU (5-Fluoruracil), Irinotecan and Oxaliplatin. Firstly, the western-blot analysis revealed that AGR2 and POLR2A are universally expressed in PDAC cell lines as well as in HPED (human pancreatic duct epithelial) cells (**Figure 6a**). The present inventors tested the effect of NTAIYY (SEQ ID NO:2) on human PDAC cell lines carrying wild-type (Capan2 and HPAC) and mutated (Panc1) TP53. Irrespective of TP53 status, the treatment of bioactive hexapeptide (NTAIYY (SEQ ID NO:2)) consistently reduced the active levels of POLR2A (POLR2A^{ser2/5}) in these cell lines (**Figure 6b**). However, it induced p53 activation in PDAC cell lines with wild-type TP53, but not in PDAC cell lines with mutated TP53. In line, two bioactive hexapeptides (NTAIYY (SEQ ID NO:2) and PTTIYY (SEQ ID NO:4)) increased the therapeutic effects of 5-FU, Irinotecan and Oxaliplatin *in vitro* in PDAC cell lines with wild-type TP53 (**Figure 6c**), as compared to controls (saline and NTAIYA (SEQ ID NO:5)). However, they seemed not to affect the therapeutic effect of Gemcitabine (**Figure 6c**). Finally, the similar results were obtained using the subcutaneous xenografts of Capan2 and HPAC cells in which the bioactive hexapeptide (PTTIYY (SEQ ID NO:4)) enhanced the cytostatic effect of 5-FU and Oxaliplatin *in vivo* (**Figure 6d, 6e**). However, no such effect was observed in the xenografts of Panel cells (**Figure 6d, 6e**).

### Example 8

### Bioactive hexapeptides intensify the cytotoxicity of α-Amanitin in human PDAC cell lines with wild-type TP53

α-Amanitin is the most potent and specific inhibitor of RNAPII known so far, which slows down the translocation rate of RNAPII on DNA; thus, the present inventors hypothesize that the bioactive hexapeptide intensifies the cytotoxic effect of α-Amanitin by hindering the nuclear import of catalytic subunit of RNAPII. To test this, the present inventors treated four PDAC cell lines with bioactive (inactive) hexapeptides in combination with α-Amanitin. Here, the treatment of both bioactive hexapeptides (PTTIYY (SEQ ID NO:4) and NTAIYY (SEQ ID NO:2)) significantly enhanced the cytotoxic effect of α-Amanitin *in vitro* (**Figure 7a**). Again, this effect is only limited to those cell lines with wild-type TP53 (Capan2 and HPAC cells). No such effect was observed in PDAC cell lines with mutated TP53 (Panc1 and Su86.86, **Figure 7b**). Western blot analysis confirmed this synergistic effect between bioactive hexapeptides and α-Amanitin in that the combinational therapy effectively inhibited the levels of active POLR2A, but dramatically induced p53 activation and cleaved-caspase3 expression (C-C3, **Figure 7c**). Next, the present inventors tested the cytotoxic effect of bioactive hexapeptides and α-Amanitin on patient-derived PDAC organoids carrying either wild-type (B34 and B29) or mutated (B61 and B77) TP53 (**Figure 7d**). These organoids were positive for AGR2 and POLR2A expression (**Figure 7d**). Compellingly, the addition of bioactive hexapeptides (PTTIYY (SEQ ID NO:4) and NTAIYY (SEQ ID NO:2)) to α-Amanitin is lethal to TP53 wild-type organoids. As such, a very low dose of α-Amanitin alone (0.01 µg/ml) had only a marginal cytostatic effect; however, an addition of bioactive hexapeptides to this small dose of α-Amanitin eliminated the organoid growth (**Figure 7e, 7f**). Also, the treatment of bioactive hexapeptides alone partially inhibited the growth of organoid. However, no such dramatic effect was observed in PDAC organoids carrying mutated TP53 (**Figure 7e, 7f**). Due to the hepatic toxicity, the clinical application of α-Amanitin is limited. To overcome this, the present inventors used α-Amanitin conjugated to a monoclonal partially humanized anti-EpCAM antibody (Ama-HEA125). EpCAM is a cancer antigen which is overexpressed by PDAC. Next, the present inventors investigated the anti-tumour activity of Ama-HEA125 (30 µg/kg) and bioactive hexapeptides (8 µg/kg) in orthotopic xenograft tumour models established by Capan2, HAPC and Panc1 cells. Compared to control-treated (including NTAIYA (SEQ ID NO:5)) animals, the treatment of Ama-HEA125 or bioactive hexapeptide (PTTIYY (SEQ ID NO:4) and NTAIYY (SEQ ID NO:2)) alone only partially inhibited the tumour volume in the xenograft tumours generated by Capan2 and HPAC cells; however, the combinational treatment reduced the tumour volume more effectively (**Figure 7g**), verifying the results in patient-derived PDAC organoids. No such effect was observed in the xenograft tumours established by Panc1 cells (**Figure 7g**).

### Example 9

### Bioactive hexapeptides intensify the cytotoxicity of α-Amanitin in other human cancer entities

Next, the present inventors went on to test if the above described hexapeptides are also bioactive in other human cancer entities. To this end, the present inventors firstly investigated the protein expression of AGR2, p53 and POLR2A in several human cancer cell lines including colon (HCT119, SW480 and HT29), lung (A549, H2228 and H2087) and breast (McF7 and MDA-MB-231) cancers. Except for one colon cancer line (SW480), all other cell lines show the comparable levels of AGR2 and POLR2A as human PDAC cell lines (Capan2 and HPAC cells, **Figure 8a**). Also, the cancer cell lines with TP53 mutation (HT29, H2087 and MDA-MB-231) express p53 at a higher level than those with wild-type TP53 (**Figure 8a**). Consistently, the treatment of hexapeptide (NTAIYY (SEQ ID NO:2)) reduced the levels of active POLR2A in a dose-dependent manner across all tested cell lines, and it activated p53 pathway in cancer lines with wild-type TP53 (**Figure 8b**). Furthermore, the present inventors treated six (two for each cancer entity) cell lines with bioactive (inactive) hexapeptides in combination with α-Amanitin. Similar to the results obtained from PDAC cell lines, the treatment of bioactive hexapeptides (PTTIYY (SEQ ID NO:4) and NTAIYY (SEQ ID NO:2)) dramatically enhanced the cytotoxic effect of α-Amanitin *in vitro (***Figure 8c**). Western blot analysis confirmed this synergistic effect between bioactive hexapeptides and α-Amanitin in that the combinational therapy effectively triggered cleaved-caspase3 expression in cell lines with wild-type TP53 (HCT116, A549 and Mcf7, **Figure 8d**).

## Claims

1. A peptide having an amino acid sequence NTXIYY (SEQ ID NO:1), wherein X = A (SEQ ID NO:2) or T (SEQ ID NO:3).

2. The peptide according to claim 1, wherein said peptide is a hexapeptide, heptapeptide, octapeptide, nonapeptide or decapeptide.

3. The peptide according to any of claims 1 - 2, wherein X is A.

4. The peptide according to any of claims 1 - 3, wherein said peptide is a hexapeptide and has an amino acid sequence NTAIYY (SEQ ID NO:2).

5. The peptide according to any of the foregoing claims, further comprising an N-terminal protecting group and/or a C-terminal protecting group.

6. The peptide according to claim 5, wherein the N-terminal protecting group has the general formula -C(O)-R, wherein R is selected from the group consisting of H, unsubstituted or substituted alkyls, and unsubstituted or substituted aryls, and wherein the C-terminal protecting group is -NR₁R₂, R₁ and R₂ being independently selected from H and C₁-C₁₀ alkyl, said -NR₁R₂ thus forming an amide group at the C-terminus of said peptide; wherein, preferably, R is selected from the group consisting of methyl, ethyl, propyl, isopropyl, butyl and isobutyl, wherein, more preferably, the N-terminal protecting group is an acetyl group, and wherein, more preferably, the C-terminal protecting group is NH₂.

7. The peptide according to any of the foregoing claims, wherein said peptide is one of the following:
- encapsulated into a liposome;
- linked to or encapsulated into a nanoparticle; or
- encapsulated into an extracellular vesicle, which, preferably is an exosome.

8. The peptide according to claim 7, wherein
- said peptide is encapsulated into a liposome, and wherein said liposome comprises a phospholipid selected from phosphatidylcholine, phosphatidylethanolamine, phosphatidylinositol, phosphatidylserine, phosphatidic acid, phosphoinositides, phosphatidylinositol monophosphate, phosphatidylinositol bisphosphate, phosphatidylinositol triphosphate, ceramide phosphorylcholine, ceramide phosphorylethanolamine, ceramide phosphoryllipid and mixtures of any of the foregoing; or
- wherein said peptide is linked to a nanoparticle, and wherein said nanoparticle is a solid nanoparticle, preferably selected from gold (Au) nanoparticles, silica nanoparticles, and carbon nanotubes; or
- wherein said peptide is encapsulated into a nanoparticle and wherein said nanoparticle is a polymeric nanoparticle comprising a polymeric shell surrounding a core, wherein, preferably, said shell comprises one or more of the following polymers: poly(lactic acid), poly(lactic-co-glyclic acid), chitosan, gelatin, poly-alkyl-acyanoacrylate, and mixtures of the foregoing, and said peptide is contained within said core of said polymeric nanoparticle; or
- wherein said peptide is encapsulated into an extracellular vesicle, which, preferably is an exosome, and said extracellular vesicle, preferably said exosome is produced from a suitable cell/cell line.

9. The peptide according to any of claims 7 and 8, wherein said liposome additionally comprises a sterol, preferably selected from cholesterol, sitosterol, stigmasterol, stigmastanol, campesterol, fucosterol, brassicasterol, ergosterol, 9,11-dehydroergosterol, daucosterol, and esters of any of the foregoing.

10. A composition comprising a peptide according to any of claims 1 - 9, an anti-cancer agent, and a pharmaceutically acceptable carrier.

11. The composition according to claim 10, wherein said anti-cancer agent is selected from alkylating agents, platin analogues, intercalating agents, antibiotics, inhibitors of mitosis, taxoids, topoisomerase inhibitors, RNA polymerase inhibitors, antimetabolites, wherein, preferably, said anti-cancer agent is coupled to a targetting moiety, in particular an antibody, antibody fragment, a growth factor or growth factor receptor, such as EGF or EGFR.

12. The composition according to claim 11, wherein
- said alkylating agents are selected from cyclophosphamide, busulfan, carmustine, procarabzine and dacarbazine;
- said platin analogues are selected from oxaliplatin, cisplatin, carboplatin, and satraplatin;
- said intercalating agents are selected from anthracyclins, in particular doxorubicin, daunorubicin, epirubicin, idarubicin; mitoxantron, and amsacrin;
- said inhibitors of mitosis are selected from vincristin, vinblastin, vindesin and vinorelbin;
- said taxoids are selected from paclitaxel, cabazitaxel and docetaxel;
- said topoisomerase inhibitors are selected from camptothecin, topotecan, irinotecan, etoposide and teniposide;
- said RNA polymerase inhibitors are selected from amatoxins, in particular α-amanitin; and
- said antimetabolites are selected from pyrimidine analogues, in particular 5-fluoruracil, gemcitabin and cytarabine; purine analogues, in particular mercaptopurine, 6-thioguanine, azathioprin and fludarabin; and folic acid antagonists, in particular methotrexyate and pemetrexed.

13. The peptide according to any of claims 1 - 9 or the composition according to any of claims 10 - 12, for use as a medicament.

14. The peptide or composition for use according to claim 13, in a method for preventing development or growth of a cancer tumour in a patient, and/or in a method of treating a cancer tumour in a patient, said cancer tumour being **characterised by** having a wildtype-version of the *TP53* gene, and not having a mutated version of the *TP53* gene, wherein, in said method, said peptide or composition is administered to a patient having or suspected of having or developing a cancer tumour.

15. The peptide or composition for use according to claim 14, wherein said cancer tumour is a pancreatic cancer tumour, a breast cancer tumour, a colon cancer tumour or a lung cancer tumour, wherein, preferably, said cancer tumour is a pancreatic cancer tumour which is selected from pancreatic adenocarcinoma tumour, pancreatic acinar cell carcinoma tumour, and pancreatic cystadenocarcinoma tumour, wherein, more preferably, said cancer tumour is a pancreatic ductal adenocarcinoma (PDAC) tumour.

16. The peptide for use according to claim 13, in a method of sensitising a cancer tumour to the action of an anti-cancer agent in a patient, wherein, in said method, said peptide is administered to a patient having or suspected of having or developing a cancer tumour, and wherein said anti-cancer agent is administered to said patient concomitantly with said administration of said peptide.


SEQUENCE LISTING

<110> Klinikum rechts der Isar der Technischen Universität München

<120> Peptides for the treatment of cancer

<130> K31865EP

<160> 9

<170> BiSSAP 1.3.6

<210> 1
<211> 6
<212> PRT
<213> Artificial Sequence

<220>
<223> SEQ ID NO:1

<220>
<221> VARIANT
<222> 3
<223> Xaa is A or T

<400> 1
Asn Thr Xaa Ile Tyr Tyr
1               5

<210> 2
<211> 6
<212> PRT
<213> Artificial Sequence

<220>
<223> SEQ ID NO:2

<400> 2
Asn Thr Ala Ile Tyr Tyr
1               5

<210> 3
<211> 6
<212> PRT
<213> Artificial Sequence

<220>
<223> SEQ ID NO:3

<400> 3
Asn Thr Thr Ile Tyr Tyr
1               5

<210> 4
<211> 6
<212> PRT
<213> Artificial Sequence

<220>

<223> SEQ ID NO:4

<400> 4
Pro Thr Thr Ile Tyr Tyr
1               5

<210> 5
<211> 6
<212> PRT
<213> Artificial Sequence


<220>
<223> SEQ ID NO:5

<400> 5
Asn Thr Ala Ile Tyr Ala
1               5

<210> 6
<211> 5
<212> PRT
<213> Artificial Sequence


<220>
<223> SEQ ID NO:6

<400> 6
Thr Ala Ile Tyr Tyr
1               5

<210> 7
<211> 30
<212> PRT
<213> Mus musculus


<220>
<223> Polr2a sequence

<400> 7
Leu Glu His Thr Thr Leu Arg Lys Val Thr Ala Asn Thr Ala Ile Tyr
1               5                   10                  15
Tyr Asp Pro Asn Pro Gln Ser Thr Val Val Ala Glu Asp Gln
            20                  25                  30

<210> 8
<211> 30
<212> PRT
<213> Homo sapiens


<220>
<223> Polr2a sequence

<400> 8
Leu Glu His Thr Thr Leu Arg Lys Val Thr Ala Asn Thr Ala Ile Tyr
1               5                   10                  15
Tyr Asp Pro Asn Pro Gln Ser Thr Val Val Ala Glu Asp Gln
            20                  25                  30

<210> 9

<211> 5
<212> PRT
<213> Artificial Sequence

<220>
<223> SEQ ID NO: 9

<220>
<221> VARIANT
<222> 2
<223> Xaa is any amino acid

<220>
<221> VARIANT
<222> 3
<223> Xaa is I or L

<220>
<221> VARIANT
<222> 4
<223> Xaa is Y or F

<220>
<221> VARIANT
<222> 5
<223> Xaa is Y or F

<400> 9
Thr Xaa Xaa Xaa Xaa
1               5